# EUROPÄISCHE PATENTANMELDUNG

(11) **EP 2 154 133 A1**
(43) Veröffentlichungstag der Anmeldung: **17.02.2010**
(21) Anmeldenummer: 08162407.4
(22) Anmeldetag: 14.08.2008
(51) Int. Cl.: C07D 311/34, C07D 311/38

(54) **Synthese von Equol**

(71) Anmelder: System Biologie AG, 8808 Pfäffikon SZ (CH)
(72) Erfinder: Steffan, Bert, 50999 Köln (DE)
(74) Vertreter: Steglich, Gregor

(57) **Zusammenfassung**

Gegenstand der Erfindung ist ein Verfahren zur Herstellung von Isoflavanen aus Isoflavonen, wobei in einem ersten Reaktionsschritt (a) die 4-Ketogruppe des I-soflavons enantioselektiv und unter Erhalt der 2,3-Doppelbindung zu der 4-Hydroxyverbindung reduziert wird.

## Beschreibung

Gegenstand der Erfindung ist ein Verfahren zur Herstellung von Isoflavanen aus Isoflavonen.

Isoflavone, auch Isoflavonoide genannt, sind meist gelblich gefärbte Verbindungen, die als Derivate des Isoflavons zur Klasse der Flavonoide zählen. Isoflavone sind sekundäre Pflanzenstoffe die u. a. eine Funktion für pflanzliche Abwehr von Pathogenen ausüben. Der Grundkörper Isoflavon kommt in Kleearten vor. Einige bekanntere Isoflavone sind Daidzein, als Glucosid Daidzin in Sojamehl, Genistein aus Sojabohnen und Rotklee, Prunetin aus der Rinde von Pflaumenbäumen, Biochanin A aus Kichererbsen und Klee, Orobol, Santal aus Sandelholz, Rotholz u. a. Hölzern und Pratensein aus frischem Rot- oder Wiesenklee.

Das Isoflavon Daidzein (4',7-Dihydroxyisoflavon; 7-Hydroxy-3-(4-hydroxyphenyl)-4H-1-benzopyran-4-on) kommt in Soja vor und ist damit ein Bestandteil vieler Nahrungsmittel und Nahrungsergänzungsmittel. Das Isoflavan Equol (4',7-Dihydroxyisoflavan; 3-(4-Hydroxyphenyl)-7-chromanol) wird aus Daidzein nach der Einnahme in der Darmflora gebildet. Equol gehört somit der Gruppe der sekundären Pflanzenstoffe an. Es wird vermutet, dass die Umwandlung durch Streptokokken, Milchsäurebakterien und Bifidobakterien erfolgt. Equol ist nach dem Verzehr von Daidzein-reichen Lebensmitteln wie Soja in Blut und Harn nachweisbar. Equol besitzt eine leichte Estrogenaktivität (0,1% der Aktivität von Steroid-Estrogenen) und kann an die Estrogenrezeptoren ERα und ERβ binden. Nur etwa ein Drittel (kaukasische Bevölkerung) bis die Hälfte (Japaner) der Menschen können aus Daidzein Equol bilden. Bei den "Equolbildnern" ist der Cholesterin senkende und entzündungshemmende Effekt einer sojareichen Ernährung höher als bei Personen, die kein Equol bilden können. Vor allem für S-Equol wurden verschiedene und in Studien dokumentierte antiproliferative Einflüsse gezeigt, zum Beispiel auf Gewebeveränderungen in der Brust, die bei Frauen in den Wechseljahren auftreten können. Durch Wechselwirkung mit der 5α-Reduktase hemmt Equol bei Männern die DHT Produktion. Es wird vermutet, dass DHT bei Männern ursächlich bei der Entstehung von Prostata-Krebs verantwortlich ist.

Da Equol erst im Darm aus Daidzein entsteht, kann es nicht aus Naturprodukten isoliert werden und muss künstlich hergestellt werden. Verschiedene Publikationen beschreiben mikrobiologische Herstellungswege. So betrifft EP1025850 eine Zusammensetzung, in der Equol mikrobiologisch aus Isoflavonen aus Soja hergestellt wird. Mikrobiologische Herstellungsprozesse weisen jedoch allgemein Nachteile auf. So sind sie relativ störanfällig, weil sich Mikroorganismen verändern und dadurch veränderte Produkte erzeugen können. Die Herstellung muss daher kontinuierlich überwacht werden. Gegebenenfalls müssen die Mikroorganismen nach der Herstellung getötet und abgetrennt werden.

Daher wurde versucht, Equol im Wege der organischen Synthese herzustellen.

Muthyala et al., 2004 beschreiben ein Verfahren zur Herstellung von Equol aus Daidzein. Dieses umfasst eine Reduktion der 2,3 DB und der Ketogruppe mittels Palladiumhydroxid-Katalysator [Pd(OH)₂], Es wird ein Enantiomerengemisch erhalten, das chromatographisch (HPLC) in R-Equol und S-Equol aufgetrennt wird.

Heemstra et al., 2006, beschreiben eine asymetrische Synthese des Chroman-Rings mittels Evans-Alkylierung und intramolekularer Buchwald-Veretherung.

Die WO 2007/016423 A2 offenbart ein Verfahren mit einer Reduktion der 2,3 Doppelbindung und der 4-Ketogruppe, Eliminierung der 4-OH-Gruppe unter Ausbildung einer 3,4 Doppelbindung, Synthese eines speziellen geeigneten Iridium-Katalysators und anschließende enantioselektive Reduktion der 3,4- Doppelbindung mit dem Iridiumkatalysator.

Gharpure et al., 2008, beschreiben ein Verfahren zur Totalsynthese von verschiedenen Isoflavanen und Equol.

Die vorteilhaften Wirkungen von Isoflavonen und Phytoestrogenen wie Equol werden üblicherweise erzielt, wenn eine Einnahme über einen längeren Zeitraum mit signifikanten Mengen erfolgt, Da der Equol-Vorläufer Daidzein in vergleichsweise großen Mengen in Soja enthalten ist, könnte eine Verabreichung von Equol als Nahrungsergänzungsmittel erfolgen. Daher ist es in hohem Masse von Bedeutung, dass der Wirkstoff auf einfache Weise, in großen Mengen und kostengünstig verfügbar ist.

Die bekannten Verfahren der organischen Synthese weisen jedoch den Nachteil auf, dass sie relativ aufwändig sind und spezielle und teure Reagenzien erfordern. Eine einfache und kostengünstige Herstellung, welche die Versorgung breiter Bevölkerungsschichten mit einer täglichen Dosis ermöglichen würde, ist mit diesen Synthesen nicht möglich. Die bekannten Verfahren eignen sich nicht zur kostengünstigen industriellen Fertigung, da weitgehend teure Edelmetallkatalysatoren oder spezielle, schwer herstellbare chirale Verbindungen als Katalysatoren oder Schutzgruppen eingesetzt werden. Außerdem erfordern die bekannten Verfahren im allgemeinen eine Enantiomerentrennung, was den Arbeitsaufwand und daher auch die Kosten der industriellen Herstellung deutlich erhöht.

### Problem:

Das der vorliegenden Anmeldung zugrunde liegende Problem ist die Bereitstellung eines Verfahrens zur einfachen und kostengünstigen Herstellung von Isoflavanen, insbesondere von Equol. Das Verfahren soll möglichst wenige Reaktionsschritte erfordern. Der Einsatz nur aufwändig herstellbarer und teurer Chemikalien, insbesondere von Edelmetallen wie Platin und von Spezialreagenzien, wie komplexen chiralen Iridiumverbindungen, soll vermieden werden. Das Verfahren soll auf einfache Weise die Synthese unterschiedlicher Isoflavane und von Derivaten des Equol ermöglichen.

### Lösung:

Die Aufgabe wird überraschenderweise gelöst durch Verfahren mit den Merkmalen der Patentansprüche 1 bis 9.

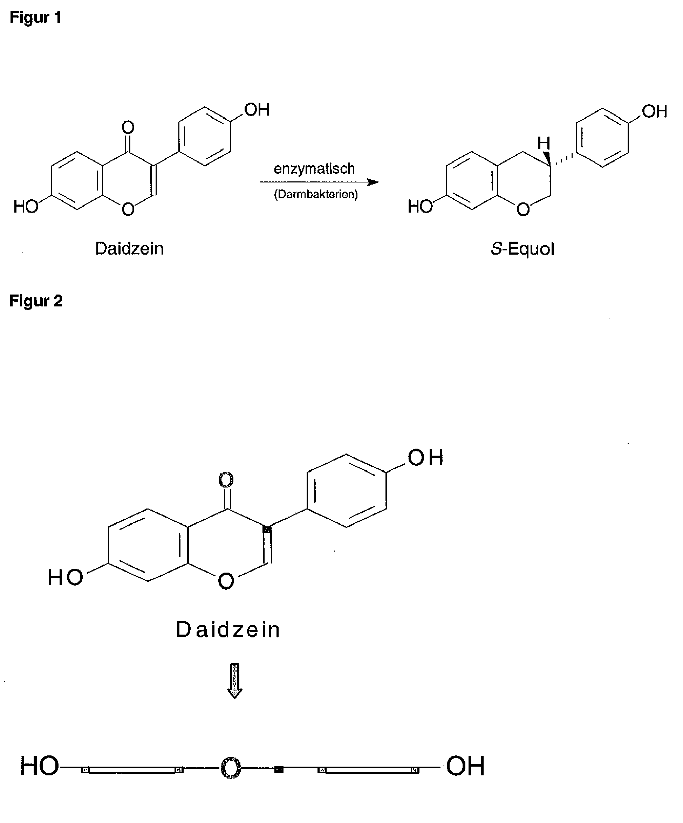

Figur 1 zeigt schematisch die Reaktion von Daidzein zu S-Equol.
Figur 2 zeigt den Ausgangsstoff Daidzein oben als Strukturformel und unten um 90° gekippt. In dieser Ansicht zeigt die 4-CO-Gruppe auf den Betrachter.
Figur 3 zeigt eine Seitenansicht des reduzierten Daidzeins mit einer Schutzgruppe an der 4'-Position. Die 4-RO-Schutzgruppe blockiert die Oberseite des Moleküls und erlaubt die Reduktion nur von der Unterseite.

Das neue Verfahren beruht auf der folgenden Überlegung: Durch Blockierung der Vorder- oder Rückseite des an der 4-Position stereoselektiv hydrierten Isoflavons, insbesondere mittels Veretherung oder Veresterung der 4-Hydroxygruppe, wird der *cis-*Angriff eines Reduktionsmittels zur Reduktion der 2,3-Doppelbindung sterisch gelenkt. Der Angriff des Reduktionsmittels erfolgt je nach Stellung der 4-RO-Gruppe an der Vorder- oder Rückseite. Die Reaktion kann dadurch auf einfache Weise unter Verwendung preiswerter Katalysatoren und Reduktionsmittel erfolgen.

Bei dem erfindungsgemäßen Verfahren zur Herstellung von Isoflavanen aus Isoflavonen wird in einem ersten Reaktionsschritt (a) die 4-Ketogruppe des Isoflavons enantioselektiv und unter Erhalt der 2,3-Doppelbindung zu der 4-Hydroxyverbindung reduziert Die erhaltene Hydroxyverbindung ist 3-(4-Hydroxyphenyl-) -4,7-dihydroxy-chromen-2. Je nach Wahl der Reaktionsbedingungen wird die chirale Verbindung 3-(4-Hydroxyphenyl-)-4(S)-hydroxy-,7-hydroxy-chromen-2 oder 3-(4-Hydroxyphenyl-)-4(R)-hydroxy-,7-hydroxy-chromen-2 erhalten. Das chirale Kohlenstoffatom der 4-Hydroxyverbindung ist das C-Atom an der 4-Position. Als Reaktionsprodukt wird die 4R- oder 4S-Form des 4-Hydroxyderivats der Ausgangsverbindung erhalten. Die Reaktion erfolgt selektiv an der 4-Position des Moleküls. Das übrige Molekül bleibt im Wesentlichen unverändert. Durch diesen ersten Reaktionsschritt wird bereits in einem frühen Stadium der Synthese ein chirales Zwischenprodukt erhalten. Die Chiralität des Moleküls bleibt bei der weiteren Umsetzung zu dem Isoflavan erhalten, so dass eine aufwändige Enantiomerentrennung vermieden wird. Ausgehend von dem Reaktionsprodukt aus Schritt (a) kann daher in einer Folgereaktion ein chirales 3S- oder 3R-Isoflavan mit einem chiralen 3C-Atom erhalten werden. Die Hydoxyverbindungen sind somit erfinderische Zwischenprodukte für die Synthese. Sie können gegebenenfalls isoliert und bei Bedarf weiter verarbeitet werden.

Die Reduktion (a) erfolgt unter Verwendung geeigneter enantioselektiver Reduktionsmittel. Dem Fachmann sind solche Mittel bekannt. Geeignete Reduktionsmittel werden beispielsweise in March's Advanced Organic Chemistry: Michael B. Smith, Jerry March 2007, Verlag Wiley, beschrieben. In bevorzugten Ausführungsformen erfolgt die Reduktion enzymatisch oder mit LiAlH(O-tert-Bu), 9-BBN oder Alpine Boran. Dabei steht 9-BBN steht für 9-Borabicyclo[3.3.1]nonan und Alpine Boran steht für B-3-pinanyl-9-borabicyclo[3.3.1]nonan.

Die in Schritt (a) erhaltene chirale 4S- oder 4R- Hydroxyverbindung wird bevorzugt in einem zweiten Reaktionsschritt (b) weiterverarbeitet. Der Reaktionsschritt (b) kann ohne weitere Reinigungsschritte im selben Reaktionsansatz erfolgen, gegebenenfalls nach Neutralisation und/oder Entfernung von überschüssigen reaktiven Komponenten. Es ist jedoch auch möglich, die Hydroxyverbindung zunächst aufzureinigen.

In einem bevorzugten zweiten Reaktionsschritt (b) wird die enantiomere 4-Hydroxyverbindung mit einer Schutzgruppe versehen. Die 2,3-Doppelbindung bleibt erhalten Vorzugsweise ist die Reaktion (b) eine Veretherung oder Veresterung. Die Hydroxygruppe an der 4-Position im Molekül wird dabei selektiv zu einer Ether- oder Estergruppe umgewandelt. Bei der Reaktion (b) werden regelmäßig auch die weiteren Hydroxygruppen im Molekül oder zumindest ein Teil dieser Hydroxygruppen mit der Schutzgruppe versehen. In einer weiteren Ausführungsform erfolgt die Reaktion selektiv nur an der 4-Position.

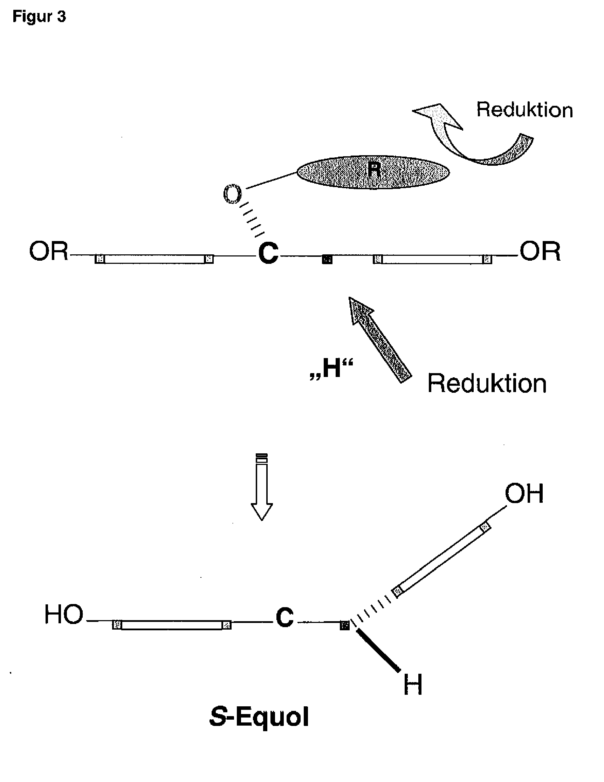

Die Schutzgruppe blockiert je nach Stellung der Hydroxygruppe die Ober- oder Unterseite des Moleküls, so dass anschließend der Angriff eines Reduktionsmittels in einem Reaktionsschritt (c) ausschließlich oder im Wesentlichen von der nicht blockierten Seite erfolgt (siehe Figur 3). Als Ergebnis dieser enantioselektiven Reduktion wird je nach vorgegebener Stereochemie der 4-RO-Gruppe nach Abspaltung der Schutzgruppe das (3R)- oder (3S)- Isoflavan als Produkt erhalten.

Bevorzugt ist die Schutzgruppe ein raumfüllender Substituent. Raumfüllende Substituenten weisen häufig verzweigte Alkylketten oder Ringstrukturen auf, die wenigstens einen aliphatischen oder aromatischen Ring enthalten. Die Ringe sind insbesondere Benzyl-, Hexyl- oder Norbornylringe. Die verzweigten Alkylketten weisen bevorzugt mindestens 4, insbesondere 4 bis 30 oder 6 bis 20 Kohlenstoffatome auf. Erfindungsgemäß geeignete Schutzgruppen für Alkoholgruppen sind dem Fachmann bekannt und können entsprechend ausgewählt werden (siehe beispielsweise Greene's Protective Groups in Organic Synthesis; Peter G. M. Wuts, Theodora Greene, 2006 Wiley Verlag). Geeignete Schutzgruppen sind beispielsweise 2-Methoxy-ethoxymethyl (MEM)-, Benzyl-, *tert*-Butyl-, Toluylsulfonyl- und Silyl, insbesondere Trimethylsilylgruppen.

Das Reaktionsprodukt aus Schritt (b), also das an der 4-Position geschützte 4S- oder 4R-Hydroxyderivat des Eduktes, wird bevorzugt in einem dritten Reaktionsschritt (c) umgesetzt, bei dem die 2,3-Doppelbindung der geschützten 4-Hydroxyverbindung reduziert wird. Bei der Reaktion wird gleichzeitig die geschützte 4-Hydroxygruppe reduziert. Dabei wird natürlich auch die 4-Schutzgruppe entfernt. Es entsteht ein Isoflavan. Dieses weist ein chirales C-Atom an der 3-Position auf. Je nach Wahl der Ausgangsverbindung und der Reduktionsmittel kann so ein 3S- oder 3R-Isoflavan erhalten werden.

Die enantioselektive Reduktion der 2,3-Doppelbindung kann mittels einfacher, üblicher und kostengünstiger Katalysatoren erfolgen. Es ist nicht erforderlich, chirale Katalysatoren einzusetzen. Vielmehr ist durch die Schutzgruppe eine Seite des Moleküls abgedeckt, so dass das Reduktionsmittel gezielt von der anderen Seite angreifen kann. Die Hydrierung der Doppelbindung erfolgt somit in *cis*-Anordnung nur auf einer Seite des Moleküls (siehe Figur 3). Die Reaktion erfolgt selektiv an der 2- ,3- und 4-Position des Moleküls. Die geschützte 4-Hydroxygruppe und die 2,3-Doppelbindung werden zu einer Alkylgruppe reduziert. Die weiteren Positionen des Moleküls werden nicht reduziert.

Sofern weitere Hydroxygruppen im Molekül, beispielsweise die 4'- und 7-Hydroxygruppe, geschützt wurden, können diese bei der Reaktion (c) ebenfalls abgespalten werden. Sofern weitere Schutzgruppen bei der Reaktion (c) nicht entfernt werden, so werden sie gegebenenfalls in einer weiteren Reaktion abgespalten. Eine solche zusätzliche Abspaltung der verbleibenden Schutzgruppen kann beispielsweise mit LiAlH₄ oder nach weiteren bekannten Methoden erfolgen (siehe beispielsweise Greene's Protective Groups in Organic Synthesis; Peter G. M. Wuts, Theodora Greene, 2006 Wiley Verlag).

Für die Reduktion (c) können dem Fachmann bekannte übliche Reduktionsmittel eingesetzt werden, die selektiv die Doppelbindung und die substituierte 4-Alkoholgruppe reduzieren. Solche Reduktionsmittel werden beispielsweise in March's Advanced Organic Chemistry: Michael B. Smith, Jerry March, 2007, Wiley Verlag. beschrieben. In bevorzugten Ausführungsformen der Erfindung erfolgt Schritt (c) durch Hydroborierung mit anschließender Protolyse, durch Addition von Diamiden, durch Reduktion mit BINAP, Wilkinson-Katalysatoren, Vaska-Katalysator, Silanen oder durch enzymatische Reduktion, insbesondere mit Enoyl-ACP-Reduktase.

Die Hydroborierung ist eine Reaktion der organischen Chemie. Die Hydroborierung von Alkenen mit Boran liefert ein Alkylboran. Dieses Zwischenprodukt wird *in situ* durch Protolyse zu dem Alkan weiterverarbeitet Die Hydroborierung kann beispielsweise mit 9-BBN und die Protolyse beispielsweise mit einem Protonendonator wie Ameisensäure oder Propionsäure erfolgen.

Ein bevorzugtes Diamid ist Hydrazin der Summenformel N₂H₄.

BINAP (2,2'-Bis(diphenylphosphino)-1,1'-binaphthyl) ist ein bidentater Diphosphinligand mit axialer Chiralität, der bei vielen Übergangsmetall-katalysierten Reaktionen, speziell asymmetrische Hydrierungen, außergewöhnliche Eigenschaften bezüglich Enantioselektivität und Aktivität zeigt.

Der Wilkinson-Katalysator ist ein in der organischen Chemie verwendeter Homogenkatalysator mit der Summenformel C₅₄H₄₅ClP₃Rh. Es handelt sich um einen Rhodiumkomplex, der zur Hydrierung, Hydroformylierung, Hydrosilylierung und zur Isomerisierung von Allyl-Gruppen zu Propenyl-Gruppen Anwendung findet.

Vaska-Katalysator (Vaskas Komplex) ist der Trivialname der Iridiumverbindung *trans-*Chlorocarbonylbis(triphenylphosphin)iridium(I). Dieser quadratisch-planare diamagnetische Komplex besteht aus einem zentralen Iridiumatom, an dem zwei Triphenylphosphin-Liganden zueinander *trans* gebunden sind. Des Weiteren befinden sich noch ein Chlorid und ein Carbonylligand in der Koordinationssphäre des Metalls.

Silane sind reaktive Verbindungen, die mindestens eine Si-H oder Si-R Bindung aufweisen, wobei R für einen organischen Rest steht.

Enzyme, die selektiv Doppelbindungen reduzieren, sind bekannt. Die Enoyl-ACP-Reduktase katalysiert unter NADPH-Verbrauch die Reduktion von Crotonyl-ACP zu Butyryl-ACP.

Das erfindungsgemäß hergestellt Isoflavan kann nach Ablauf der Reaktion (c) anschließend in einem Schritt (d) nach bekannten Methoden gereinigt werden wie beispielsweise Destillation, Umkristallisation, Chromatographie und/oder Filtration. Solche Reinigungsmethoden können auch nach den Reaktionsschritten (a) oder (b) eingesetzt werden. In bevorzugten Ausführungsformen folgen die Schritte (a) und (b), die Schritte (b) und (c) oder insbesondere alle drei Schritte (a) bis (c) unmittelbar aufeinander, ohne das die Zwischenprodukte gereinigt werden.

Das erfindungsgemäße Verfahren kann in wenigen einfachen Verfahrenschritten durchgeführt werden. In einer bevorzugten Ausführungsform der Erfindung besteht das Syntheseverfahren ausschließlich aus den Reaktionsschritten (a), (b) und (c). Eine Enantiomerentrennung ist nicht erforderlich. Bevorzugt umfasst das Verfahren keine Enantiomerentrennung.

Vorzugsweise wird das Endprodukt der Reaktion (c), wie auch das Zwischenprodukt der ersten Reaktion (a) oder (b), in seiner S- oder R-Form in hoher Reinheit erhalten. Insbesondere weist das Reaktionsprodukt bei Schritt (a), (b) und/oder (c) mindestens zu 70, 85 oder 90%, bevorzugt mindestens zu 95, 98 oder 99% das gewünschte R- oder S-Stereoisomer auf.

Der Einsatz von Edelmetallen wie Silber, Gold, Platin oder Palladium ist für die Durchführung des erfindungsgemäßen Verfahrens nicht erforderlich. Bevorzugt erfolgt das gesamte Verfahren ohne den Einsatz von Edelmetallen. Auch der Einsatz von chiralen Schwermetallkatalysatoren, wie Iridiumkatalysatoren, ist nicht erforderlich. Bevorzugt erfolgt das gesamte Verfahren ohne den Einsatz von chiralen Schwermetallkatalysatoren.

Als Ausgangsstoff für das erfindungsgemäße Verfahren wird bevorzugt Daidzein eingesetzt. Als natürlich vorkommendes Isoflavan ist Daidzein in großen Mengen verfügbar. Beispielsweise kann Daidzein eingesetzt werden, das aus Sojaextrakten erhalten wurde. Das erfindungsgemäße Verfahren hat daher den Vorteil, dass bereits der Ausgangsstoff kostengünstig ist. Bei der Verwendung von Daidzein als Ausgangsstoff wird als Reaktionsprodukt enantiomeres Equol erhalten. In bevorzugten Ausführungsformen der Erfindung wird die S-Form des Equol oder die R-Form hergestellt. Für die S-Form sind insgesamt mehr vorteilhafte physiologische Wirkungen bekannt.

Erfindungsgemäß können jedoch allgemein Isoflavone als Ausgangsstoffe eingesetzt werden. Es ist dabei bevorzugt, Derivate des Daidzeins einzusetzen. Als Reaktionsprodukt wird dann an Stelle von Equol ein entsprechendes Derivat des Equols erhalten. "Derivate" im Sinne der Erfindung sind beispielsweise Substanzen, die die Struktur der Stammverbindung (Daidzein oder Equol) und mindestens einen zusätzliche Substituenten aufweisen. Derivate sind insbesondere auch Verbindungen, bei denen die Hydroxygruppen nicht (nur) an der 4'- und 7-Position angeordnet sind, sondern an unterschiedlichen oder zusätzlichen Positionen, insbesondere an der 5'- und 6'-Position.

Wenn als Ausgangsstoff ein derivatisiertes Daidzein eingesetzt wird, so weist das Endprodukt (das entsprechend derivatisierte Equol) an derselben Positionen im Grundgerüst des Moleküls dieselben Substituenten auf. Der oder die Substituenten sind dabei vorzugsweise so ausgewählt, das sie die erfindungsgemäßen Reaktionen nicht oder nur unwesentlich beeinträchtigen. Dies ist im Allgemeinen unproblematisch, da übliche Substituenten wie Hydroxygruppen und Alkylreste an den beiden Benzylringen des Isoflavons die Reaktivität der 4-Ketogruppe und 2,3-Doppelbindung nicht in hohem Maße nachteilig beeinflussen.

Geeignete Substituenten des Isoflavon/Isoflavan-Grundgerüsts sind beispielsweise solche, bei denen einer der beiden Phenylringe des Grundgerüsts substituiert ist, beispielsweise mit Alkyl-, Aryl-, Araryl-, Alkoxy-, Halogen-, Hydroxy-, Nitro-, Sulfat-, Sulfonat-, Hydroxamat-, Imino- oder. Diese Substituenten können auch an dem 2C-Atom des Daidzeins vorliegen. Die Alkylsubstituenten weisen insbesondere 1 bis 10 Kohlenstoffe auf und sind verzweigt oder unverzweigt, beispielsweise Methyl, Ethyl, Propyl, Isopropyl, Butylgruppen. Die Alkyl, Aryl, Araryl und Alkoxysubstituenten können mit Heteroatomen substituiert sein, beispielsweise mit Hydroxy, Amino, Alkoxy oder Halogengruppen. Weitere geeignete Derivate des Daidzeins sind Verbindungen, bei denen eine oder mehrere Hydroxygruppen verestert oder verethert sind, insbesondere an der 4'-und/oder 7-Position. Die Derivate können auch mindestens eine Hydroxygruppe an einer unterschiedlichen Position aufweisen als an der 4'- oder 7-Position. Geeignete Derivate sind beispielsweise Daidzin, 3'-Hydroxydaidzein zur Synthese von 3'-Hydroxyequol, 2',3'-Hydroxydaidzein zur Synthese von 2',3'-Hydroxyequol und 4'-Methoxy-2'-hydroxydaidzein zur Synthese von Vestitol (4'-Methoxy-2'-hydroxyequol).

In einer weiteren bevorzugten Ausführungsform der Erfindung wird der Reaktionsschritt (c) so durchgeführt, dass ein Derivat des Isoflavans, insbesondere des Equols, erhalten wird, das an der 2C-Position substituiert ist. Dies ist beispielsweise auf einfache Weise möglich, wenn auf die Hydroborierung nicht eine Protolyse folgt, sondern eine unterschiedliche Reaktion. Auf diese Weise kann an der 2C-Position beispielsweise eine Hydroxygruppe oder Alkoxygruppe eingeführt werden.

### Ausführungsbeispiele:

### Beispiel 1: Reduktion der 4-Keto-Funktion von Daidzein (1) zur 4-(R)-Hydroxygruppe.

Zu einer Lösung von (S)-CBS-Oxazaborolidin (0,1 eq) in THF wurde eine Lösung von N-Ethyl-N-Isopropylanilin-Boran (1 eq) in THF gegeben. Zu dieser Mischung wurde langsam bei 25°C Daidzein (leq) gelöst in THF innerhalb von 1,5h zugetropft. Danach wurde die Reaktionsmischung noch 10 min. gerührt, vorsichtig mit Methanol gequencht und 30 min gerührt. Das Lösungsmittel wurde im Rotationsverdampfer entfernt und das Produkt durch Flash-Chromatographie an Silica-Gel (230-400 mesh) mit dem Eluenten Ethylacetat/Hexan (4:1 v/v)gereinigt. Die Enantiomerenreinheit wurde mittels HPLC an einer chiralen Säule zu 96 % ee bestimmt (25 cm Chiralcel OD-H Chiral Column; iso-PrOH/Hexan: 1/99; Flussrate: 0.3 mL/min; Detektion: 254 nm); Die Ausbeute betrug 78% Daidzein -4-ol (2).

### Beispiel 2: Schutz der 4, 7, 4'-Hydroxygruppen des Dihydrodaidzeins

Insbesondere die 4-Hydroxygruppe wird mit einem raumfüllenden Substituenten geschützt, der die Aufgabe hat, die R-Seite des Moleküls sterisch zu blockieren. Als Substituent wird Brombenzylbromid eingesetzt. Neben der 4-Hydroxygruppe werden auch die beiden aromatischen Hydroxygruppen verethert. 3.5 eq NaH wurden in wasserfreiem THF mit 0.35 eq p-BrC6H4CH2Br und 0.1 eq Dihydrodaidzein (2) aus Beispiel 1 in THF unter Rückfluss 12h gerührt. Die Ausbeute nach Flash-Chromatographie an Silica-Gel (230-400 mesh) mit dem Eluenten Ethylacetat/Hexan (4:1 v/v) 80% war Daidzein-4,7,4'tri-brombenzylether (3).

### Beispiel 3: Reduktion der 2,3 Doppelbindung im Chromen-System (3)mit 9-BBN.

Zu einer Lösung von Verbindung (3) (1 eq) in wasserfreiem THF tropft man bei Raumtemperatur unter Rühren und Stickstoff-Atmosphäre 1 eq 9-BBN. Nach 1h Rühren bei RT werden tropfenweise 2n Ameisensäure zugesetzt und bei max. 30° - 40° C 1h Stunde gerührt. Die Reaktionsmischung wird mit Ether verdünnt und mit Wasser 2x gewaschen. Der organische Überstand wird einrotiert und mit Flash-Chromatographie an Silica-Gel (230-400 mesh) mit dem Eluenten Ethylacetat/Hexan (4:1 v/v) gereinigt. Die Enantiomerenreinheit wurde mittels HPLC an einer chiralen Säule zu 98 % ee bestimmt (25 cm Chiralcel OD-H Chiral Column; A Ethanol/Hexan: 10/90; B Ethanol/Hexan 90/10; Gradient 15min; Flussrate: 1 mL/min; Detektion: 254 nm); Ausbeute 75%: 2, 3(S)-Dihydrodaidzein 4,7,4'tri-brombenzylether (4).

### Beispiel 4: Entfernung der Hydroxybenzylgruppe:

Zu einer Suspension von LiAlH4 in Ether gibt man Verbindung (4) gelöst in Ether tropfenweise zu und rührt unter Rückfluss für ca. 3h. Der Reaktionskolben wird mit Eiswasser abgekühlt und der Inhalt vorsichtig tropfenweise mit Eiswasser versetzt, bis die Wasserstoffentwicklung beendet ist. Anschließend wird so viel 10%ige Schwefelsäure zugegeben, bis sich der Aluminiumhydroxid-Niederschlag aufgelöst hat. Im Scheidetrichter wird die organische Phase abgetrennt und durch Flash-Chromatographie an Silica-Gel (230-400 mesh) mit dem Eluenten Ethylacetat/Hexan (4:1 v/v) gereinigt. Die Enantiomerenreinheit des Produktes wurde mittels HPLC an einer chiralen Säule zu 98 % ee bestimmt (25 cm Chiralcel OD-H Chiral Column; A Ethanol/Hexan: 10/90; B Ethanol/Hexan 90/10; Gradient 15min; Flussrate: 1 mL/min; Detektion: 254 nm), Ausbeute: 95% (S)-Equol.

### Literatur

Muthyala, R.S., Ju,Y.H., Sheng,S., Williams,L.D., Doerge,D.R., Katzenellenbogen, B.S., Helferich,W.G. and Katzenellenbogen,J.A. (2004) Equol, a natural estrogenic metabolite from soy isoflavones: convenient preparation and resolution of R- and S-equols and their differing binding and biological activity through estrogen receptors alpha and beta. Bioorg. Med. Chem., 12, 1559-1567.

Heemstra,J.M., Kerrigan,S.A., Doerge,D.R., Helferich, G.H., Boulanger,W.A. (2006) Total Synthesis of (S)-Equol. Organic Letters, 8, 5441-5443.

Gharpure, S. J., Sathiyanarayanan, A. M., Jonnalagadda, P. (2008) o-Quinone methide based approach to isoflyvans: application of the total syntheses of equol, 3'-hydroxyequol and vestitol. Tetr. lett. 49, 2974-2978.

## Patentansprüche

1. Verfahren zur Herstellung von Isoflavanen aus Isoflavonen, wobei in einem ersten Reaktionsschritt (a) die 4-Ketogruppe des Isoflavons enantioselektiv und unter Erhalt der 2,3-Doppelbindung zu der 4-Hydroxyverbindung reduziert wird.

2. Verfahren nach Anspruch 1, wobei in einem zweiten Reaktionsschritt (b) die 4-Hydroxyverbindung mit einer Schutzgruppe versehen wird.

3. Verfahren nach Anspruch 2, wobei in einem dritten Reaktionsschritt (c) die 2,3-Doppelbindung der geschützte 4-Hydroxyverbindung reduziert wird, so dass ein Isoflavan erhalten wird.

4. Verfahren nach einem der vorhergehenden Ansprüche, wobei in Reaktionsschritt (a) die Reduktion enzymatisch, mit LiAlH(O-*tert*-Bu), 9-BBN und/oder Alpine Boran erfolgt.

5. Verfahren nach einem der Ansprüche 2 bis 4, wobei die Schutzgruppe ausgewählt ist aus 2-Methoxy-ethoxymethyl (MEM)-, Benzyl-, *tert*-Butyl-, Toluylsulfonyl- und Trimethylsilylgruppen.

6. Verfahren nach einem der Ansprüche 3 bis 5, wobei der Reaktionsschritt (c) durch Hydroborierung mit anschließender Protolyse, durch Addition von Diamiden, durch Reduktion mit BINAP, Wilkinson-Katalysatoren, Vaska-Katalysator, Silanen oder enzymatisch erfolgt.

7. Verfahren nach einem der vorhergehenden Ansprüche, wobei das Isoflavon Daidzein ist.

8. Verfahren nach einem der Ansprüche 3 bis 7, wobei bei Reaktionsschritt (c) die 2,3-Doppelbindung der geschützte 4-Hydroxyverbindung zu einer C-C Einfachbindung umgesetzt wird und dabei ein Derivat des Isoflavans erhalten wird, das an der 2-Position mit einem Substituenten unterschiedlich von H versehen ist.

9. Chirale Verbindung, ausgewählt aus
3-(4-Hydroxyphenyl-)-4-(*S*)-hydroxy-,7-hydroxy-chromen-2 und
3-(4-Hydroxyphenyl-) 4-(*R*)-hydroxy-,7-hydroxy-chromen-2.
